Europäisches Patentamt

(19) European Patent Office

Office européen des brevets

(11) Publication number : **0 488 959 A2**

# EUROPEAN PATENT APPLICATION

(21) Application number : 91810921.6

(22) Date of filing : 26.11.91

(51) Int. Cl.⁵ : **A61K 31/66,** A61K 31/675,
A61K 31/00

(30) Priority : 28.11.90 GB 9025851
07.12.90 GB 9026642
07.12.90 GB 9026640

(43) Date of publication of application :
03.06.92 Bulletin 92/23

(84) Designated Contracting States :
AT BE CH DE DK ES FR GB GR IT LI LU NL SE

(71) Applicant : SANDOZ LTD.
Lichtstrasse 35
CH-4002 Basel (CH)
(84) BE CH DK ES FR GB GR IT LI LU NL SE

(71) Applicant : SANDOZ-PATENT-GMBH
Humboldtstrasse 3
W-7850 Lörrach (DE)
(84) DE

(71) Applicant : SANDOZ-ERFINDUNGEN
Verwaltungsgesellschaft m.b.H.
Brunner Strasse 59
A-1235 Wien (AT)
(84) AT

(72) Inventor : Koyuncuoglu, Eyüp Hikmet
Acibadem, Pomak Sok. 4/3
Kadiöky-Istanbul (TR)
Inventor : Urwyler, Stephan
Weissensteinweg 3
CH-3303 Jegenstorf (CH)

(54) New uses of competitive NMDA receptor antagonists.

(57) The use of competitive NMDA receptor antagonists in the treatment of withdrawal symptoms in patients undergoing drug withdrawal, for suppression of dependence, habituation or addiction on a dependence-producing drug and for preventing or inhibiting opiate tolerance.

EP 0 488 959 A2

The present invention relates to a new use, in particular new pharmaceutical use for competitive N-methyl-D-aspartate (NMDA) receptor antagonists. More particularly the invention relates to the use of competitive N-methyl-D-aspartate (NMDA) receptor antagonists in i) the treatment, especially preventive treatment of withdrawal symptoms in patients undergoing drug withdrawal from dependence-producing drugs, e.g. treatment of abstinence syndromes, ii) for the suppression of dependence, habituation or addiction in patients occasioned by dependence-producing drugs and minimizing the probability of relapses and iii) for the attenuation of tolerance to opiate type drugs associated with any long-term use of such drugs, particularly pain relief.

Competitive NMDA antagonists are characterized by a competitive mechanism of action i.e. a condition in which the agonist and antagonist bind to the same recognition sites on the NMDA receptor, and thus when present concomitantly compete for such sites.

Of particular interest are the following competitive NMDA receptor antagonists:

D-(-)(E)-4-(3-phosphonoprop-2-enyl)piperazine-2-carboxylic acid (D-CPP-ene),
S-α-amino-5-phosphonomethyl-[1,1'-biphenyl]-3-propanoic acid,
E-2-amino-4-methyl-5-phosphono-3-pentenoic acid,
E-2-amino-4-methyl-5-phosphono-3-pentenoic acid ethyl ester,
cis-4-phosphonomethyl-2-piperidinecarboxylic acid,
(R)-4-oxo-2-amino-5-phosphono-pentanoic acid,
2-amino-4,5-(1,2-cyclohexyl)-7-phosphonoheptanoic acid,
4-(phosphonomethyl)-DL-phenylglycine,
4-(3-phosphonopropyl)-2-piperidinecarboxylic acid,
2-(2-phosphonoethyl)-DL-phenylalanine,
3-carboxy-5-(phosphonoethyl)-1,2,3,4-tetrahydroisoquinoline,
3-carboxy-5-phosphono-1,2,3,4-tetrahydroisoquinoline,
cis-DL-4-[(1(2)H-tetrazol-5-yl)methyl]-2-piperidinecarboxylic acid,
cis-4-(3-phosphonoprop-1-enyl)-2-piperidinecarboxylic acid,
E-2-amino-4-propyl-5-phosphono-3-pentenoic acid,
phosphoric acid 4-(2-carboxy-piperidinyl) ester and
1-[4-(4-chloro-α,α-dimethylbenzyl)-2-methoxyphenyl]-1,2,4-triazole-3-carboxamide.

Most preferred competitive NMDA receptor antagonists include:
D-(-)(E)-4-(3-phosphonoprop-2-enyl)piperazine-2-carboxylic acid (D-CPP-ene),
S-α-amino-5-phosphonomethyl-[1,1'-biphenyl]-3-propanoic acid,
E-2-amino-4-methyl-5-phosphono-3-pentenoic acid,
E-2-amino-4-methyl-5-phosphono-3-pentenoic acid ethyl ester and

cis-4-phosphonomethyl-2-piperidinecarboxylic acid,

These compounds are described in UK Patent Application No. 2201676, UK Patent 2 198 134, European Patent Applications 233154, 203891, 364996 and 405834 US Patents 4918064, 4761405 and 4657899.

Such compounds have been proposed for a number of uses, including anticonvulsant, muscle relaxant and protectant against neuronal degeneration induced by stroke or hypoglycemia, but as of the priority date of the present invention had not been proposed for the above uses.

A dependence-producing drug as used herein is a drug having the capacity to interact with a living organism to produce a state of psychic or physical dependence or both. Such a drug may be used medically or nonmedically without necessarily producing such a state. The characteristics of a state of drug dependence, once developed, will vary with the type of drug involved. There are several types of drug that, because they can produce substantial central nervous stimulation or depression, or disturbances in perception, mood, thinking, behaviour, or motor function, are generally recognized as having the capacity, under certain circumstances of use, to produce individual and public health and social problems. As used herein the term "dependence-producing drug(s)" means drugs of the following types:

   a) alcohol type, e.g. ethanol and certain other drugs with sedative effects, such as chlordiazepoxide, diazepam, meprobamate, methaqualone,
   b) amphetamine type - e.g. amphetamine, dexamphetamine, methamphetamine, methylphenidate and phenmetrazine,
   c) cocaine type - cocaine and coca leaves,
   d) hallucinogen type - e.g. fentanyl, α-methylfentanyl, 3-methylfentanyl, etryptamine, dimethyltryptamine, and
   e) opiate type - e.g. opiates such as morphine, heroin and codeine, and synthetics with morphine-like effects, such as methadone, pethidine, meperidine.

Particular classes of drug to which competitive NMDA antagonists may be applied in withdrawal therapy, especially preventive treatment of withdrawal syndromes and for suppression of dependence, habituation or addiction include ethanol, cocaine and opiate type.

The character of the withdrawal symptoms that appear when a dependence-producing drug is discontinued depend upon many factors, including the particular drug, the total daily dose used, the interval between doses, the duration of use, and the health and personality of the user.

Dependency, habituation or addiction is in all cases characterised by a want or craving for renewed dosaging after administration has been ceased, as well as the occurrence of severe side reactions or withdrawal symptoms when on-going drug dosaging, e.g. chronic high-dose drug abuse, is abruptly discontinued, for example stereotyped abstinence syndrome, as well as individual withdrawal symptoms including lacrymation, rhinorrhoea, yawning, sweating, dilated pupil, anorexia, tremor, insomnia, sneezing, coryza, hot and cold flushes, abdominal discomfort, nausea, vomiting, abdominal cramps, diarrhoea, increased heart rate/blood pressure, increased respiratory rate and depth, muscular pain, CNS-hyperexcitability, anxiety, epileptic seizures, dysphoria, etc.

Tolerance is characterised by a decrease in the effectiveness of a given dose of a drug during repeated administration, thus requiring a progressive increase in the dose to produce a given level of effect. For example, a consequence of tolerance to opiates is a need to increase the dose of the drug to sustain the analgesic effect.

In one aspect, the present invention relates to the use of NMDA antagonists in the treatment, especially preventive treatment of withdrawal symptoms in patients undergoing drug withdrawal from dependence-producing drugs.

In accordance with the present invention it has been found that competitive NMDA receptor antagonists are useful for the treatment, especially preventive treatment of withdrawal symptoms and may thus be employed as a means enabling or facilitating withdrawal of drugs, e.g. of the various types herein above listed, where appropriate progressively, e.g. via a series of dosage reductions over a period of days or weeks, from persons addicted or habituated thereto or dependent thereon, thus making it possible to wean the treated subject fully from his dependence, habit or addiction.

The efficacy of a competitive NMDA receptor antagonist in the treatment, especially preventive treatment of withdrawal symptoms caused by terminating the long term administration of a dependence-producing drug, is demonstrated e.g. in the rat and mouse using standard pharmacological tests e.g. as described hereinafter.

The ability of a competitive NMDA antagonist to suppress ethanol withdrawal symptoms can be shown in mice at a dosage of 0.3 to 30 mg/kg i.p. according to the method of K.A. Grant et. al., Eur. J. Pharm. 176 (1990) 289-296. In this test D-CPP-ene decreases the occurrence and severity of the withdrawal seizures in a dose of 0.3-10 mg/kg i.p.

For the investigation of the effects of competitive NMDA receptor antagonists on the precipitated morphine abstinence syndrome, one pellet containing 75 mg morphine base is subcutaneously implanted on the back of mice or rats under light ether anesthesia. The animals are divided into six groups 72 hours after pellets implantation. The first group consisting of control receive physiological saline (i.p.), the second group is injected with the NMDA antagonist (0.3-30 mg/kg i.p.) in the same volume of saline. Immediately after the injections the animals are placed in an observation chamber and strictly observed for 15 min. The third and fourth groups are given i.p. physiological saline and the NMDA antagonist, respectively, and ten min following the first administration they receive 0.5 mg/kg of naloxone i.p.

The fifth and sixth groups are given i.p. saline and the NMDA antagonist respectively and 5 min. following the first administration they receive naloxone (0.5 mg/kg i.p.). After naloxone administration the animals of the third, fourth, fifth and sixth groups are placed in an observation chamber and strictly observed for 15 min.

During the observation period of 15 min the number of some countable precipitated abstinence syndrome signs such as flying (a vigorous kind of jumping), jumping, wet dog shake, writhing, teeth chattering, defecation, diarrhoea, ptosis are counted and rated.

In this test D-CPP-ene significantly decreases defecation whereas ptosis is significantly increased as compared to the saline group, when naloxone is given 10 min. after the administration of D-CPP-ene. When naloxone is given 5 min. after the administration of D-CPP-ene flying (a vigorous kind of jumping), jumping, teeth chattering and writhing is significantly higher, whereas wet dog shake and defecation are significantly decreased.

If the time between administration of the competitive NMDA antagonist and naloxone is increased e.g. 45 min., an attenuation or prevention of the morphine abstinence syndrome precipitated by naloxone injection is observed at doses of 0.3-30 mg/kg i.p.

In a further method the competitive NMDA antagonists intensify the intensity of the abstinence syndrome in rats before the development of opiate physical dependence. This is shown in the following test:

Rats are divided into 6 groups. The first group is given saline 5 times daily i.p. The second group is given a competitive NMDA antagonist (0.1-30 mg/kg i.p.), e.g. D-CPP-ene, the third group is given naloxone (2 mg/kg i.p.), the fourth group morphine (5 mg/kg i.p.) 5 times daily. To the fifth and sixth group is administered naloxone (2 mg/kg i.p.) and 10 min later either a NMDA antagonist (0.1-30 mg/kg i.p.) or morphine (5 mg/kg i.p.) respectively. The administration of the substances is continued for 5 days. On the fifth day, 1 hour before the last injection, three pellets containing 75 mg morphine base (total 225 mg) are subcutaneously implanted on the back of all the rats under light anesthesia. Three days after the pellet implantation, all the rats are given naloxone (2 mg/kg i.p.) and they are immediately placed in an observation chamber. Then the numbers of jumps, wet dog shakes, writhing, teeth chatterings and defecations are counted for 10 min and rated. Naloxone, morphine and the NMDA antagonist administered 5 times a day for 5 days before pellet implantation significantly increase the intensity of the abstinence syndrome.

A further significant increase of abstinence syndrome in the rats belonging to the fifth and sixth group, both of which receive D-CPP-ene combined with naloxone or morphine, is observed.

In this test competitive NMDA antagonists, for example D-CPP-ene show an increase of abstinence syndromes as dextromethorphane, as described in H. Koyuncuoglu et al. Pharmacol. Biochem. & Behav. 1991, 39, 575-579, the contents of which are incorporated herein by reference for which clinical efficacy has been proven in heroin addicts (E. Koyuncuoglu et al., Int. J. Clin. Pharmacol. Ther. Toxicol. 28, 147-152, 1990 the contents of which are incorporated herein by reference.

Another test the rat Social Interaction Test, is based on that described by S. E. File, J. Neurosci. Meth., 1980, 2, 219-238. In this test male rats (200-250 g), are housed 5 to a cage and kept in the laboratory environment for at least a week before testing. Rats paired in the test are taken from separate cages. The test arena consists of an open-topped box, 62 x 62 x 33 cm with a 7 x 7 matrix of infra-red photocell beams in the walls, 2.5 cm from the floor. The light intensity at the floor of the arena is 3.5 lux under low light conditions. Rats are exposed to the arena in pairs for 10 minutes on the day before the experiment. Rats are paired with different partners on the test day.

The Social Interaction Test consists of placing each member of a pair of rats in opposite corners of the arena and then leaving them undisturbed for 10 minutes while recording their behaviour remotely on videotape. The behavioural assessments are made subsequently from the recordings.

The time spent in social interaction is measured and expressed as a cumulative total for the 10 minute session. The behaviours that comprised social interaction are: following with contact, sniffing (but not sniffing of the hindquarters), crawling over and under, tumbling, boxing and grooming. Sniffing of the hindquarters is excluded because it is markedly influenced by the degree of urination and defaecation.

Thus, in the present experiment, rats (n = 8 pairs) are dosed with a dependence-producing drug, e.g. either diazepam or morphine or cocaine for 7 days. The dependence-producing drug is abruptly ceased and the rats are tested at various times e.g. 24 h, after the last dose in the above described Social Interaction Test.

NMDA antagonists are tested by subjecting both members of a pair of rats to the same treatment at the pre-determined time before testing. Where a 45 minute pre-treatment time is used, the rats are placed singly in small cages immediately after dosing until they are tested. Where longer pre-treatment times are used, the rats are returned to their home cages after dosing and placed in the single cages 45 minutes before testing.

Upon abrupt cessation of dosing with the dependence-producing drug, the rats display an abstinence syndrome manifest as a reduction in social interaction. This abstinence syndrome is reversed by administration of the test compound, 0.3-30 mg/kg i.p. 45 min. before testing.

Treatment of the rats with the test compound, 0.3-30 mg/kg i.p., alone, has no effect on social interaction under the low light familiar conditions.

The use of a competitive NMDA antagonist, in particular of any one of the Examples given above, in the treatment, especially preventive treatment, of withdrawal symptoms may be demonstrated in clinical trials, e.g. as carried out in conventional manner, e.g. in analogous manner to that described in H. Koyuncuoglu et al., Int. J. Clin. Pharmacol. Ther. Toxicol. 28, 147-152, 1990.

Competitive NMDA receptor antagonists are therefore indicated for use in the treatment, especially preventive treatment, of drug withdrawal symptoms in patients undergoing drug withdrawal from dependence-producing drugs.

For this use the appropriate dosage will, of course, vary depending upon, for example, the NMDA receptor antagonist employed, the host, the mode of administration, and the nature and severity of the condition being treated. An indicated daily dosage is in the range from about 1 to about 3000 mg, e.g. about 5 to about 800 mg of a competitive NMDA antagonist conveniently administered, for example, in divided doses up to four times a day.

For D-CPP-ene an indicated dosage is e.g. from about 50 to 3000 mg/day e.g. 50 to 300 mg/day. For S-α-amino-5-phosphonomethyl-[1,1'-biphenyl]-3-propanoic acid an indicated dosage is e.g. from about 5 to 3000 mg/day, e.g. 5 to 200 mg/day.

In accordance with the foregoing the present invention provides:

The use of a competitive NMDA receptor antagonist, in particular any one of the Examples given above, for the manufacture of the Exmples given above, for the manufacture of a medicament for the treatment, e.g. amelioration, especially preventive treatment of drug withdrawal symptoms, in a subject in need thereof, for example in a subject dependent, habituated or addicted to a drug substance, in particular any drug substance as hereinbefore set forth, and undergoing withdrawal from said drug substance.

Additionally, the invention relates to the use of competitive NMDA receptor antagonists for the suppression of dependence, habituation or addiction in patients occasioned by dependence-producing drugs.

Dependence, habituation or addiction involves a compulsive need to take a drug, to experience its psychic effects, and to avoid the discomfort of its absence.

In accordance with the present invention it also has been found that competitive NMDA receptor antagonists are useful for the suppression of dependence, habituation or addiction on a dependence-producing drug.

The effects of the competitive NMDA receptor antagonists in the suppression of dependence on a dependence-producing drug is observed in rhesus monkeys, wherein the antagonist is administered in a programmed manner to monkeys which can self-administer themselves with morphine over 4 weeks.

Monkeys are tested for their physical dependence liability according to the test described in R.W. Foote et al., Life Sciences 1988, 42, 137-152. Drug naive monkeys receive by i.v. infusion from 0.1-15 mg/kg e.g. 1 to 10 mg/kg e.g. 2 mg/kg daily, of the NMDA receptor antagonist by programmed administration, e.g. 48 infusions per day, and may self-administer morphine (100 microgram/kg per infusion per administration), also intravenously from the 2 nd week. The trial lasts for 6 weeks. A typical dose of morphine that is self-administered by animals receiving NMDA antagonist treatment is 10 to 12 mg/kg per day. This is surprisingly lower than the typical dose self-administered by control animals (e.g. about 50% lower).

The effects of the NMDA receptor antagonists in suppression of dependence is also observed in rats, wherein the antagonist is administered in a programmed manner to rats which can self-administer themselves with cocaine over several weeks.

Rats are tested for their physical dependence liability according to the test described by N.E. Goeders et al (Pharmacol. Biochem. Behaviour 33, 859-866, 1989). All rats self-administer cocaine under base-line conditions with stable rates of responding. In control animals the average intake of low dose of intravenous cocaine (0.5 mg/kg) ranges between 20-29 injections per session (3.4-4.9 mg/session), while the intake of the higher dose (1 mg/kg) ranges between 17-22 injections per session (5.8-7.5 mg/session).

In the above test treatment with the NMDA antagonists (0.3-30 mg/kg i.p.) causes a significant decrease in the intake of cocaine.

In a further test NMDA antagonists reduce alcohol consumption in alcohol preferring rats on p.o. administration of about 1 to about 200 mg/kg.

Male and female rats of an alcohol preferring strain were placed in individual cages each containing an eatometer according to D. Fallon [Science 148, 977-8 (1965)] and two bottles fitted with double sphere valves, allowing a gravimetric determination of the quantities of food and beverage actually taken. The animals received a vitamin rich standard chow, distilled water in one bottle and an aqueous ethanol solution (10 % per volume) in the other. Under these conditions 50 to 97 % of the absorbed liquid was taken from the bottle containing the ethanol solution, which corresponds to a daily consumption of 4 to 6 g pure ethanol by kg animal body weight, and the rats developed an ethanol addiction which appeared from their increasing startle behaviour when shaking the cages during the abstinence periods.

The compounds were administered p.o. in aqueous solution and the consumption of food, water and alcohol was recorded during 12 hours and compared with the average consumption within a period of 3 days before the administration.

In this test, the alcohol consumption is significantly reduced by the administration of competitive NMDA receptor antagonists without significant reduction of food and liquid ingestion.

Competitive NMDA receptor antagonists are therefore useful in the suppression of dependence, habituation or addiction on a dependence-producing drug in subjects dependent, habituated or addicted to such drugs (whether consequential to drug abuse or as a result of regular, e.g. long-term, chronic clinical use), for example to morphine addicts.

The compounds are therefore also indicated for use in reducing the craving for a drug after addiction to that drug and can therefore be used in maintenance therapy during remission from addiction to drugs. The compounds may also be used for prophylactic treatment of subjects liable to become dependent on drugs. The compounds are also useful to minimize the probability of relapses.

For this use the appropriate dosage will, of course vary depending upon, for example, the NMDA receptor antagonist employed, the host, the mode of administration and the nature and severity of the condition being treated. An indicated daily dosage is in the range from about 1 to about 3000 mg, e.g. about 5 to about 800 mg of a competitive NMDA receptor antagonist conveniently administered, for example, in divided doses up to four times a day.

For D-CPP-ene an indicated dosage is e.g. from about 50 to 3000 mg/day, e.g. 50 to 300 mg/day. For S-α-amino-5-phosphonomethyl-[1,1′-biphenyl]-3-propanoic acid an indicated dosage is e.g. from about 5 to 3000 mg/day, e.g. 5 to 200 mg/day.

In accordance with the foregoing the present invention provides:

The use of a competitive NMDA receptor antagonist, in particular any one of the Examples given above, for the manufacture of a medicament for the suppression of dependence, habituation or addiction on a dependence-producing drug and for minimizing the relapses in a subject in need of such treatment.

In another aspect the invention relates to the use of competitive receptor antagonists for inhibiting opiate tolerance.

In accordance with the present invention it has been found that competitive NMDA antagonists attenuate the development of tolerance to the analgesic effect of opiate type drugs used in pain relief. This can be shown in the following test:

Sprague-Dawley male rats receive saline (1 ml/kg i.p.) or a competitive NMDA antagonist (0.1-10 mg/kg i.p.) followed 30 min later by saline or morphine (10 mg/kg s.c.). The injections are administered twice daily for 10 consecutive days. Analgesic effect is assessed by the tail flick test (F.E. D'Armour and D.L. Smith, J. Pharmac. Exp. Ther. 72, 74, 1971) 1 hour after the morphine injection. The analgesic response to morphine in saline treated rats shows a development of tolerance. Rats treated with competitive NMDA antagonists, e.g. D-CPP-ene, show less tolerance.

The competitive NMDA antagonists are therefore indicated for use in inhibiting opiate tolerance in pain therapy.

For this use the appropriate dosage will, of course, vary depending upon, for example, the NMDA receptor antagonist employed, the host, the mode of administration and the nature and severity of the condition being treated. An indicated daily dosage is in the range from about 1 to about 3000 mg, e.g. about 5 to about 800 mg of a competitive NMDA antagonist conveniently administered, for example, in divided doses up to four times a day.

For D-CPP-ene an indicated dosage is e.g. from about 50 to 3000 mg/day e.g. 50 to 300 mg/day. For S-α-amino-5-phosphonomethyl[1,1'-biphenyl]-3-propanoic acid an indicated dosage is e.g. from about 5 to 3000 mg/day e.g. 50 to 200 mg/day.

In accordance with the foregoing the present invention provides:

The use of a competitive NMDA receptor antagonist, in particular any one of the Examples given above, for the manufacture of a medicament for preventing or inhibiting opiate tolerance in a subject in need of such treatment.

The NMDA receptor antagonist may be administered by any conventional route, in particular enterally, preferably orally e.g. in the form of tablets or capsules, or parenterally, e.g. in the form of injectable solutions or suspensions.

A unit dosage may contain from about 0.25 to about 1500 mg of a NMDA receptor antagonist.

The pharmaceutical compositions can be prepared according to conventional techniques.

## Claims

1. Use of a competitive NMDA receptor antagonist for the manufacture of a medicament for treating drug withdrawal symptoms.

2. The use of claim 1 wherein the treatment is preventive.

3. The use of claim 1 wherein the drug is ethanol.

4. Use of a competitive NMDA receptor antagonist for the manufacture of a medicament for preventing morphine abstinence syndrome.

5. Use of a competitive NMDA receptor antagonist for the manufacture of a medicament for suppressing dependence, habituation or addiction on a dependence-producing drug.

6. Use of a competitive NMDA antagonist in the manufacture of a medicament for preventing or inhibiting opiate tolerance.

7. The use of anyone of claims 1 to 6 wherein said NMDA receptor antagonist is
D-(-)(E)-4-(3-phosphonoprop-2-enyl)piperazine-2-carboxylic acid,
S-α-amino-5-phosphonomethyl-[1,1'-biphenyl]-3-propanoic acid,
E-2-amino-4-methyl-5-phosphono-3-pentenoic acid,
E-2-amino-4-methyl-5-phosphono-3-pentenoic acid ethyl ester,
cis-4-phosphonomethyl-2-piperidinecarboxylic acid,
(R)-4-oxo-2-amino-5-phosphono-pentanoic acid,
2-amino-4,5-(1,2-cyclohexyl)-7-phosphonoheptanoic acid,
4-(phosphonomethyl)-DL-phenylglycine,
4-(3-phosphonopropyl)-2-piperidinecarboxylic acid,
2-(2-phosphonoethyl)-DL-phenylalanine,
3-carboxy-5-(phosphonoethyl)-1,2,3,4-tetrahydroisoquinoline,
3-carboxy-5-phosphonomethyl-1,2,3,4-tetrahydroisoquinoline,
cis-DL-4-[(1(2)H-tetrazol-5-yl)methyl]-2-piperidinecarboxylic acid,
cis-4-(3-phosphonoprop-1-enyl)-2-piperidinecarboxylic acid,
E-2-amino-4-propyl-5-phosphono-3-pentenoic acid,
phosphoric acid 4-(2-carboxy-piperidinyl) ester and
1-[4-(4-chloro-α,α-dimethylbenzyl)-2-methoxyphenyl]-1,2,4-triazole-3-carboxamide.

8. The use of anyone of claims 1 to 6 wherein said NMDA receptor antagonist is D-(-)(E)-4-(3-phosphonoprop-2-enyl)piperazine-2-carboxylic acid, S-α-amino-5-phosphonomethyl-[1,1'-biphenyl]-3-propanoic acid, E-2-amino-4-methyl-5-phosphono-3-pentenoic acid, E-2-amino-4-methyl-5-phosphono-3-pentenoic acid ethyl ester, cis-4-phosphonomethyl-2-piperidinecarboxylic acid.

9.  The use of anyone of claims 1 to 6 wherein said NMDA receptor antagonist is D-(-)(E)-4-(3-phosphonoprop-2-enyl)piperazine2-carboxylic acid.